(19) Europäisches Patentamt
European Patent Office
Office européen des brevets

(11) Publication number: **0 256 586 B1**

(12) # EUROPEAN PATENT SPECIFICATION

(45) Date of publication of patent specification: **13.05.92**

(51) Int. Cl.[5]: **C07C 233/01**, C07C 233/00, C12P 1/00, C07D 301/02, C07C 213/00

(21) Application number: **87201434.5**

(22) Date of filing: **24.07.87**

(54) **Process for the preparation of esters of 4-(2,3-epoxypropoxy)phenylacetic acid and 4-(2-hydroxy-3-isopropylamino-propoxy)phenylacetic acid and/or atenolol in stereospecific form.**

(30) Priority: **28.07.86 GB 8618324**

(43) Date of publication of application:
**24.02.88 Bulletin 88/08**

(45) Publication of the grant of the patent:
**13.05.92 Bulletin 92/20**

(84) Designated Contracting States:
**AT BE CH DE ES FR GB GR IT LI LU NL SE**

(56) References cited:
**EP-A- 0 166 527**
**EP-A- 0 193 227**
**FR-A- 2 250 752**
**GB-A- 2 046 259**

(73) Proprietor: **GIST-BROCADES N.V.**
**Wateringseweg 1**
**NL-2611 XT Delft(NL)**

Proprietor: **SHELL INTERNATIONALE RESEARCH MAATSCHAPPIJ B.V.**
**Carel van Bylandtlaan 30**
**NL-2596 HR Den Haag(NL)**

(72) Inventor: **Phillips, Gareth Thomas**
**5, The Finches**
**Sittingbourne Kent(GB)**
Inventor: **Bertola, Mauro Attilio**
**A. van Scheltemaplein 91**
**NL-2624 PJ Delft(NL)**
Inventor: **Marx, Arthur Friedrich**
**Fl. Nightingalellaan 12**
**NL-2614 GM Delft(NL)**
Inventor: **Koger, Hein Simon**
**G. Vermootenstraat 32**
**NL-2064 XR Spaarndam(NL)**

(74) Representative: **Huygens, Arthur Victor, Dr. et al**
**c/o Gist-Brocades N.V. Patent & Trademarks Department Martinus Nijhofflaan 2 PO Box 1**
**NL-2600 MA Delft(NL)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.08/2.19/2.0)

## Description

The present invention relates to a process for the preparation of atenolol (4-(2-hydroxy-3-isopropylamino-propoxy)phenylacetamide) in a stereospecific form and to pharmaceutically acceptable salts thereof, for example acid addition salts, and to the production of esters of 4-(2,3-epoxypropoxy)phenylacetic acid and 4-(2-hydroxy-3-isopropylamino-propoxy)phenylacetic acid in stereospecific form.

Many biologically active compounds are synthesized as a mixture of stereoisomers. Such mixtures are frequently used as such in agricultural and pharmaceutical applications. Usually more of the desired biological activity resides in only one stereoisomer so that where the mixture contains two or more stereoisomers, the potency of the mixture is reduced. A major reason for the continued use of mixtures of stereoisomers is that the cost of separation of the stereoisomers exceeds the potential advantage of a possible increase in activity. However, it is apparent that modern pharmacologists are becoming increasingly aware of other implications of the administration of mixtures wherein one or more stereoisomers have to be regarded not only merely as impurities that may not have the desired therapeutic effect, but even may have other unwanted physiological effects including toxicity.

Some examples are cited below to illustrate the association of biological activity with a single stereoisomer.

Within the pharmaceutical area, most of the beta-adrenergic blocking agents are presented as mixtures although the activity resides mainly in one stereoisomer. In one instance, a drug known as labetalol has a combined alpha-adrenergic blocking and beta-adernergic blocking action that has been shown to be attributed to two separate pairs of isomers from the mixture of four. N. Toda et al. reported in J.Pharmacol.Exp.Ther. 207 (1978) 311 that (-)-metoprolol is 270 to 380 times more potent than (+)-metoprolol in attenuating the response of rabbit atria and tracheal muscles to isoproterenol (a beta-adrenoreceptor stimulant).

Current routes to single stereoisomer beta-blockers generally involve chemical resolutions or rather lengthy chemical syntheses from stereoisomeric precursors, which are for example described in US patent 4,408,063 an in J.Org.Chem. 41 (1976) 3121 by L.M. Weinstok et al.. These processes to prepare analogously the S enantiomer (optically active stereoisomer) of metoprolol are not economically advantageous in industrial applications. Therefore an object of the present invention is to provide an efficient process for the preparation of certain stereoisomers which may be carried out on an industrial scale in an economically attractive way.

The ability of micro-organisms to convert stereospecifically short-chain alkenes ($C_2$-$C_4$) into their corresponding epoxyalkanes in a gas/solid or in a two-liquid phase bioreactor is demonstrated by J. Tramper et al. (3rd European Congress on Biotechnology, München, 10-14 September 1984). Another example of a microbiological preparation of epoxyalkanes is disclosed in the US patent 4,106,986 describing the conversion of straight-chain 1-alkenes ($C_2$-$C_{20}$) into 1,2-epoxyalkanes. In European patent application EP-A-0099609 examples are given of the conversion of propene and 1-octene into their corresponding epoxyalkanes. However, the conversion of substituted alkenes, such as 4-allyloxyphenylacetates, can in no way be deduced from these known processes, which are only applicable to straight and sometimes branched alkenes.

European patent application EP-A-0166527 describes a process for the preparation of epoxides and, more particularly, of 2,3-epoxypropyl ethers from the corresponding allyl ethers by means of micro-organisms. However, in this application, the epoxidation is only demonstrated for eight phenylallyl ethers, only three of which could lead to beta-adrenergic blockers.

Moreover, the optical purity of the epoxy compounds was low and varied between 71% and 79% depending on the epoxide and the micro-organism used. Therefore the resulting product still needs the separation in both enantiomers.

The present invention provides a stereo-selective epoxidation process which comprises subjecting an ester of 4-allyloxyphenylacetic acid to the action of a Pseudomonas oleovorans strain having the ability of stereo-selective epoxidation so as to produce the corresponding ester of 4-(2,3-epoxypropoxy)phenyl acetic acid which is predominantly in S configuration.

By predominantly in the S configuration, we mean that more than 50% by weight of the resulting epoxypropoxy compound has S configuration and in practice, our invention enables at least 70% by weight, preferably at least 80% by weight and particularly at least 90% by weight of the resulting epoxypropoxy compound to be produced having S configuration.

The epoxypropoxy compound having predominantly S configuration can be converted into atenolol by methods known per se which maintain the desirable S configuration in the propoxy side-chain. Although, in principle, any ester of 4-allyloxyphenylacetic acid can be used in the process of the invention, practical

considerations indicate that the ester will normally be a lower alkyl ester, that is containing up to 6 and normally up to 4 carbon atoms and methyl or ethyl esters are to be preferred. The ester of the epoxypropoxyphenylacetic acid can then be reacted with isopropylamine to give the corresponding ester of 4-(2-hydroxy-3-isopropylamino-propoxy)phenylaceticacid and this ester in turn can be amidated by reaction with ammonia to form atenolol. The resulting atenolol can then be converted, if desired, into pharmaceutically acceptable salts by reaction with a suitable acid by methods known per se.

Preferably the process is carried out in such a way, by selecting a suitable Pseudomonas oleovorans strain, that of the atenolol at least 70%, preferably at least 80% and more preferably at least 90% by weight is formed in the S configuration.

The Pseudomonas oleovorans strains may advantageously be immobilized for example on a polymer gel. This can be done with living cells, killed cells and/or resting cells, alternatively with suitable enzymes derived therefrom, which may be purified to a certain extent if a higher specific activity is needed.

Therefore by the term Pseudomonas oleovorans strains or substances derived therefrom" is meant the Pseudomonas oleovorans strains, killed, alive or resting, and extracts therefrom, optionally concentrated and/or purified. For example, enzymes optionally in combination with, for example, artificial or natural cofactors, may be used. No fermentatively active cells may be used for the epoxidation of the ester of 4-allyloxyphenylacetic acid. It was found that enzymes derived from living cells or killed cells may produce the S-isomer under suitable conditions. The Pseudomonas oleovorans strains or substances derived therefrom may be used several times and are generally active for at least 2 weeks. Even without a co-substrate (for example glucose) the Pseudomonas oleovorans strains may remain active. The enrichment in S-isomer of the ester of 4-(2,3-epoxypropoxy)phenylacetic acid may take place in suitable buffers as well as in physiological salt solutions. After being stored the induced cells were found to be directly capable to produce the enrichment in S-isomer.

Particularly preferred Pseudomonas oleovorans strains for the epoxidation of 4-allyloxyphenylacetates include Pseudomonas oleovorans which has been deposited with the ATCC under the accession number of 29347 on June 21, 1976, or a variant or mutant thereof.

In practising a preferred embodiment of the process of the present invention, a Pseudomonas oleovorans strain having the ability to convert 4-allyloxyphenylacetates into 4-(2,3-epoxypropoxy)-phenylacetates, at least 70% and preferably at least 80% and more preferably at least 90% by weight of which having the S configuration may be selected, and may be cultured for 0.5 to 10 days. Hereafter the cells may be suspended in a liquid nutrient medium, and the 4-allyloxyphenylacetate is subjected to the action of the cells. Alternatively the cells may be killed, for example suspended in a lysis medium and the 4-allyloxyphenylacetate is subjected to the action of the substances derived from the cells.

After this cultivation, for about 0.5 to 10 days, the cells may be isolated from the culture medium before suspending them in the liquid nutrient medium or suspending the cells in a lysis medium. To grow the Pseudomonas oleovorans strains used for the stereo-selective epoxidation of the 4-allyloxyphenylacetate, ordinary culture media containing an assimilable carbon source (for example glucose, glycerol, lactate, hydrocarbons like hexane (C6), etc.), a nitrogen source (for example ammonium sulphate, ammonium nitrate, ammonium chloride, etc.), and an inorganic nutrient source (for example phosphate, magnesium, potassium, zinc, iron and other metals in trace amounts) may be used. A preferred culture medium is a PSX medium optionally enriched with one or more additives. Optionally, an inducer (for example diethoxymethane) may be added to the culture medium. A temperature of 0 to 45°C and a pH of 3.5 to 9 is preferably maintained during the growth of the Pseudomonas oleovorans strains. Preferably the Pseudomonas oleovorans strains are grown at a temperature of 20 to 37°C and at a pH of 5 to 8.

The aerobic conditions required during the growth of the Pseudomonas oleovorans strains can be provided according to any of the well established procedures, provided that the supply of oxygen is sufficient to meet the metabolic requirement of the Pseudomonas oleovorans strains. This is most conveniently achieved by supplying oxygen, preferably in the form of air. During the conversion of 4-allyloxyphenylacetate into 4-(2,3-epoxypropoxy)phenylacetate, the Pseudomonas oleovorans strains might be in a growing stage using an abovementioned ordinary culture medium. The Pseudomonas oleovorans strains may be supplemented with a co-substrate.

During the conversion of 4-allyloxyphenylacetate into 4-(2,3-epoxypropoxy)phenylacetate, the Pseudomonas oleovorans strains can be held in a growing stage or in a substantially non-growing stage using a minimal culture medium. As minimal culture medium, an ordinary culture medium may be used containing an assimilable carbon source when required (for example glucose, ethanol, hydrocarbons like hexane (C6), etc.) a nitrogen source when required (for example ammonium sulphate, ammonium nitrate, ammonium chloride, etc.), and an inorganic nutrient source when required (for example phosphate, magnesium, potassium, zinc, iron and other metals in trace amounts). The Pseudomonas oleovorans strains

can be kept in the non-growing stage for example by exclusion of the assimilable carbon source or the nitrogen source. A preferred culture medium is a PSX medium optionally enriched with one or more additives. A temperature of 0 to 45° C and a pH of 3.5 to 9 is preferably maintained during this stage. Preferably the Pseudomonas oleovorans strains are kept at a temperature of 30 to 45° C and a pH of 5 to 8. The aerobic conditions required during this stage can be provided according to the above-mentioned procedures, provided that the supply of oxygen is sufficient to meet the metabolic requirement of the Pseudomonas oleovorans strains but also to convert 4-allyloxyphenylacetate into 4-(2,3-epoxypropoxy)-phenylacetate.

The 4-(2,3-epoxypropoxy)phenylacetate produced by the Pseudomonas oleovorans strains as mentioned above, can be recovered and purified according to any of the well established procedures.

R-(-)-4-(2,3-epoxypropoxy)phenylacetate, S-(+)-4-(2,3-epoxypropoxy)phenylacetate or mixtures thereof produced according to the present invention can be converted into R-(+)-atenolol, S-(-)-atenolol or mixture thereof, respectively, by the subsequent chemical reactions with isopropylamine and ammonia. An example of the reaction to form beta-adrenergic receptor blocking agents with the absolute (S)-configuration from aryl glycidyl ethers with the absolute (S)-configuration is described in published German patent application DE-A-2453324. The chemical conversion of S or R methyl-4-(2 hydroxy-3-isopropylaminopropoxy)-phenylacetate into S or R-atenolol was accomplished using either aqueous ammonia or anhydrous ammonia. The former conditions whilst giving S-atenolol starting from S methyl-4-(2-hydroxy-3-isoproylaminopropoxy)phenylphenylacetate in low yield gave a high proportion of the free acid arising from the hydrolysis of the ester, whereas the use of anhydrous ammonia afforded the required product in good yield. Atenolol may be converted into a pharmaceutically acceptable salt thereof like an acid addition salt.

Therefore a process for the production of atenolol according to the invention comprises reacting methyl-4-(2-hydroxy-3-isopropylaminopropoxy)phenylacetate with ammonia and at least partly separating atenolol and/or converting atenolol into the pharmaceutically acceptable salt thereof.

Especially mixtures with a predominant amount of the compound S (-)-atenolol can be advantageously used in pharmaceutical products. Preferably those compounds having at least 80% and more preferably at least 90% by weight the S configuration can be used in pharmaceutical products.

Productivities of ca. 25 mg/g/h over 3 h and product levels of 1.3 g/l can be obtained using Pseudomonas oleovorans for epoxidising 4-allyloxyphenylacetate. Moreover by using 4-allyloxyphenylacetate as substrate with glucose as co-substrate even higher productivities are possible, and productivities of up to 94 mg/g/h and product levels of 4.1 g/l were achieved over 3 h. The resulting products were enantiomerically pure within experimental error.

The optical purity as will be mentioned in this specification is represented as percent enantiomeric excess: S-R/S + R.

The present invention will be further illustrated by the following Examples without restricting the scope to these Examples.

Example 1

Transformation of methyl-4-allyloxyphenylacetate to methyl-4-(2,3-epoxypropoxy)phenylacetate using **Pseudomonas oleovorans** ATCC 29347.

A pregrown culture of Pseudomonas oleovorans ATCC 29347 was used to innoculate PSX medium pH 7.0 containing 0.75% glycerol and 0.05% diethoxymethane. PSX contains: $KH_2PO_4$ (8.92 g/l), $Na_2HPO_4$ - (2.94 g/l), $(NH_4)_2HPO_4$ (1.0 g/l), $(NH_4)_2SO_4$ (0.2 g/l), KCl (0.2 g/l), $Na_3$citrate (0.294 g/l), $CaSO_4.2H_2O$ (0.005 g/l), $MgSO_4.7H_2O$ (0.2 g/l), and PS II trace elements solution (10 ml/l). (PS II trace elements solution contains:
$(NH_4)_2SO_4.FeSO_4.6H_2O$ (0.25 g/l), $ZnSO_4.7H_2O$ (0.05 g/l), $MnCl_2.4H_2O$ (0.03 g/l), $CuSO_4.5H_2O$ (0.015 g/l), $CoCl_2.6H_2O$ (0.015 g/l), $H_3BO_4$ (0.005 g/l), $Na_2MoO_4.2H_2O$ (0.0055 g/l, KI (0.01 g/l), HCl to pH 3).

The culture (15 litres) was grown at 30° C for 24 h. The cells were recovered, resuspended in PSX medium (1.5 litres) containing 0.5% glucose. Aliquots of 100 ml were incubated with 2.5% methyl 4-allyloxyphenylacetate for up to six hours. Samples (1 ml) were extracted with methylene chloride at designated time intervals and assayed for methyl S-(+)-4-(2,3-epoxypropoxy)phenylacetate by gaschromatographic analysis (glc).

Gas chromatographic analysis

Analysis was performed on a Varian 3700 instrument using a column of 3% OV1 on WHP 100-120 (50 cm x 2mm).

Conditions: $N_2$ at 30 °C/min, Flame Ionisation Detector at 280 °C, injector at 200 °C. Programmed at 120-190 °C at 10 °C/min. The retention time of methyl-4-allyloxyphenylacetate is 1.5 min and of methyl-4-(2,3-epoxypropoxy)phenylacetate 3.1 min.
The results are given in Table 1.

Table 1

| Conversion of methyl 4-allyloxyphenylacetate into methyl S-(+)-4-(2,3-epoxypropoxy)phenylacetate using Pseudomonas oleovorans ATCC 29347 | | | | |
|---|---|---|---|---|
| Biomass g/l | S-(+)-methyl-4-(2,3-epoxypropoxy)phenylacetate mg/g/h (g/l) | | | |
| | t = 1 h | t = 3 h | t = 4 h | t = 6 h |
| 16.3 | 48 (0.8) | 78 (3.8) | 71 (4.6) | 58 (5.6) |
| 14.6 | 77 (1.1) | 94 (4.1) | 72 (4.2) | 49 (4.3) |
| 15.5 | 53 (0.8) | 69 (3.2) | 68 (4.2) | 55 (5.1) |

Example 2

Transformation of methyl 4-allyloxyphenylacetate into methyl **S**-(+)-4-(2,3-epoxypropoxy)phenylacetate using **Pseudomonas oleovorans** ATCC 29347 and its subsequent conversion into methyl **S**-(-)-4-(2-hydroxy-3-isopropylaminopropoxy)phenylacetate

PSX mineral salts medium 15 l, pH 7.0, containing 0.75% glycerol and 0.05% diethoxymethane was inoculated with a pregrown liquid culture of Pseudomonas oleovorans ATCC 29347 and incubated for 24 h at 30 °C on an orbital shaker. The cells were harvested by centrifugation and resuspended in PSX medium pH 7.5 (1.5 l). Aliquots of 100 ml were placed in 2.0 l shake flasks and incubated with 0.5% glucose and 2.5% methyl 4-allyloxyphenylacetate for 6 h at 37 °C. After 6 h the cells were extracted into dichloromethane (2.5 l), dried over anhydrous sodium sulphate and the solvent removed by evaporation to yield 42.7 g product containing 26% methyl S-(+)-4-(2,3-epoxypropoxy)phenylacetate. The crude isolate was purified over silica gel (550 g) eluted with ether/hexane: 4/6 to yield methyl-4-(2,3-epoxypropoxy)-phenylacetate (11.04 g), $[\alpha]_D^{25}$ = +8.27 ° (c = 1.39, EtOH). The pmr ($CDCl_3$) gave signals at δ 2.72/2.90

(2d, 2H, 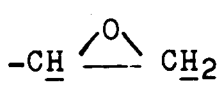), 3.32 (m, 1H,

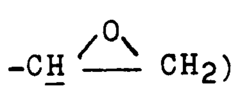),

3.95/4.18 (2d, 2H, $OCH_2$), 6.88/7.2 (2d, 4H, Aromatic), 3.57 (s, 2H, $CH_2$-CO), 3.68 (s, 3H, $OCH_3$). $Eu(hfc)_3$ pmr no separation of the signals at δ 2.72/2.90 indicating the presence of only one enantiomer. E.I. mass spectra m/z 222.

The pmr analyses were performed using a Brucker WM instrument, the optical measurements were carried out using an Optical Activity Ltd. AA 100 polarimeter.

Synthesis of methyl **S**-(-)-4-(2-hydroxy-3-isopropylaminopropoxy)phenylacetate.

To a solution of methyl S-(+)-4-(2,3-epoxypropoxy)phenylacetate (6.0 g, 27 mmole) in dry methanol (100 ml) was added isopropylamine (37 ml, 405 mmole). The reaction mixture was stirred for 24 h at 22 °C. The excess reagent and solvent were removed by evaporation and the oily residue dissolved in dichloromethane (100 ml). The dichloromethane was washed with 0.2 N HC1 (2 x 75 ml), the acid phases combined and washed with dichloromethane (2 x 50 ml) and made basic with 2N NaOH. The precipitate was extracted into dichloromethane (5 x 100 ml), dried over anhydrous sodium sulphate and the solvent evaporated to yield a semi-solid product (8.73 g). The product was recrystallised from ethylacetate/hexane to yield methyl S-(-)-4-(2-hydroxy-3-isopropylaminopropoxy)phenylacetate (6.08 g) as white crystals, m.p. 87.5-89.5 °C (uncorrected), $[\alpha]_D^{25}$ = -4.8° (C = 1.01, EtOH), pmr ($CDC1_3$) gave signals at $\delta$ = 1.08 (d, 6H, $CH(CH_3)_2$), 2.82 (m, 1H, $CH(CH_3)_2$), 2.7/2.88 (m, 2H, $CH_2NH$), 3.98 (m, 1H, CHOH), 3.94 (m, 2H, $OCH_2$), 6.88/7.2 (2d, 4H, Ar), 3.55 (s, 2H, $CH_2CO$), 3.68 (s, 3H, $OCH_3$). E.I. mass spectra m/z = 282 (m + l).

Determination of enantiomeric purity

Synthesis of the 2-oxazolidone derivatives of methyl-4-(2-hydroxy-3-isopropylamino propoxy)phenylacetate) (see J. Hermansson, J. Chromatography p. 379, **325** (1985).

(+)- or (-)-methyl-4-(2-hydroxy-3-isopropylaminopropoxy)phenylacetate (1 mg) was placed in a 1.0 ml reaction and dissolved in dry diethyl ether (0.5 ml). 0.5 N NaOH (0.05 ml) was added and the mixture stirred at room temperature for 1 minute. Phosgene (0.05 ml of a 120 mg/ml toluene solution) was added and the mixture stirred at room temperature for a further 1 h. The phases were allowed to separate, the ether phase removed, and the aqueous phase washed with dry diethyl ether (3 x 0.5 ml). The ether phases were combined and the solvent removed by evaporation under dry $N_2$. The residue was dissolved in chloroform/hexane (5/95, 2.0 ml) prior to analysis by HPLC.

The enantiomeric purity of methyl S-(-)-4-(2-hydroxy-3-isopropylaminopropoxy)phenylacetate was confirmed following its conversion into the oxazolidone derivative, which was analysed using a Gilson HPLC system (spectrophotometric detection method) on a Spherisorb S5 chiral column (UMIST packing, N-formyl-L-isoleucine covalently bound to silica, [0.25 m x 4.5 mm], Phase Separations) using a mobile phase of propan-2-ol/dichloromethane/hexane (0.75/15/84.25, v/v/v). Compound detected at 229 nm.

The analysis on the S-(-)-4-(2-hydroxy-3-isopropylamino propoxy)phenylacetate showed the compound to be enantiomerically pure (retention time 12,6 min) within experimental error (retention time of the R-isomer was 11.9 min).

Synthesis of S-(-)-4-(2-hydroxy-3-isopropylaminopropoxy)phenylacetamide (Atenolol).

Methyl-S-(-)-4-(2-hydroxy-3-isopropylaminopropoxy)phenylacetate (500 mg, 1.8 mmole) was dissolved in methanol (5.0 ml). Ammonia (1.0 ml, 35% aqueous solution) was added and the reaction allowed to proceed at 22 °C for 8 days. The excess reagent and solvent were removed by evaporation under reduced pressure and the residue dissolved in dichloromethane (10 ml). The solution was washed with 1% $NaHCO_3$ (2 x 10 ml) to remove acid, the basic washes combined and washed with dichloromethane (3 x 10 ml). The organic phases were combined, dried over anhydrous sodium sulphate and the solvent evaporated to yield crude product (0.27 g) containing the amide and starting material. The crude product was purified over neutral alumina (15 g) using a dichloromethane/methanol elution gradient. Methyl-4-(2-hydroxy-isopropylaminopropoxy)phenylacetate and atenolol were separated and analyzed on a Lichrosorb RP8 (0.25 x 4.5 mm, Hichrom) using a mobile phase of 2% ammonium acetate (pH 3.7) in methanol in a Gilson HPLC system (spectrophotometric detection system). Compounds detected at 254 nm. Retention times were 6.5 min and 3.2 min respectively. The fractions shown to contain S-(-)-Atenolol (by HPLC) were combined and the solvent evaporated to give a product which on recrystallisation from ethylacetate/hexane gave S-(-)-Atenolol (50 mg, 0.19 mmole) mp 149.5-151 °C (uncorrected), $[\alpha]_D^{25}$ = -2.82° (c = 0.332, EtOH), pmr ($CDC1_3$) gave signals at $\delta$ 1.08 (d, 6H, $CH(CH_3)_2$), 2.82 (m, 1H, $CH(CH_3)_2$), 2.7/2.88 (m, 1H, $CH_2NH$), 3.98 (m, 1H, CHOH), 3.98 (m, 2H, $OCH_2$), 6.92/7.2 (2d, 4H, Ar), 3.52 (s, $2H_2$, $CH_2CO$), 5.38 (s, 2H, $CONH_2$). EI mass spectra m/z = 267 (m + 1).

Determination of enantiomeric purity.

Diastereomeric derivatization of atenolol with (R,R)-O,O-dibenzoyl tartaric acid anhydride.

(+)- or (-)-Atenolol (2.5 mg) was placed in a 1.0 ml reaction vial. Trichloroacetic acid (0.3 ml of a solution of trichloroacetic acid (61 mg) in dry dichloroethane (6 ml)) was added and the mixture was stirred for 5 minutes at room temperature. Dibenzoyl tartaric acid anhydride (7 mg) was added and the mixture was stirred at 50°C for 3 h. 0.2 ml reaction mixture was removed, the solvent evaporated and the residue dissolved in 1.0 ml methanol for analysis by HPLC.

Analysis was performed on a Spherisorb RP 18 (0.25 m x 4.9 mm) using a mobile phase of 2% ammonium acetate (pH 3.7) in methanol (35/65, v/v) on a Gilson HPLC system using spectrophotometric detection methods. Compounds detected at 254 nm.

The enantiomeric purity was ascertained as 100% following chromatography of the diastereoisomeric derivative formed with (R,R)-O,O-dibenzoyltartaric-acid anhydride. Racemic atenolol is separated equally into its diastereoisomeric pairs when analysed in the same way with retention times of 4.4 min and 6.7 min for the R and S enantiomer respectively.

Example 3

Synthesis of S-(-)-4-(2-hydroxy-3-isopropylaminopropyloxy)phenyl acetamide [(S)-atenolol]

1.83 g of methyl (S)-(-)-4-(2-hydroxy-3-isopropylaminopropyloxy)phenyl acetate were mixed with 8 ml of 11 N anhydrous ammonia in methanol and sealed in a glass tube with a magnetic stirrer while cooling in a dry ice-acetone mixture. Thereafter, the tube was placed in an oil bath of 50°C and stirred for three days.

The tube was then cooled in a dry ice-acetone mixture, opened and the contents evaporated to give 1.74 g of a yellow solid. This was crystallized from 80 ml of hot ethyl acetate to yield 1.38 g of (S)-atenolol; m.p. 150-151.5°C; $[\alpha]_D^{25}$ 5.2° (c = 1; ethanol).

The enantiomeric purity was determined according to the determination method described in Example 2, and was ascertained as 100% following chromatography of the diastereoisomeric derivative formed with (R,R)-O,O-dibenzoyltartaric acid anhydride. Racemic atenolol is separated equally into its diastereoisomeric pair when analysed in the same way.

**Claims**

**1.** A stereo-selective epoxidation process which comprises subjecting an ester of 4-allyloxyphenylacetic acid to the action of a Pseudomonas oleovorans strain having the ability for stereo-selective epoxidation so as to produce the corresponding ester of 4-(2,3-epoxypropoxy)-phenylacetic acid which is predominantly in S configuration.

**2.** A process according to claim 1 in which more than 80% by weight of the epoxypropoxyphenylacetic acid ester has S configuration.

**3.** A process according to claim 2 in which more than 90% by weight of the epoxypropoxyphenylacetic acid ester has S configuration.

**4.** A process according to claim 1 wherein the micro-organism is Pseudomonas oleovorans (ATCC 29347) or a mutant or variant thereof.

**5.** A process according to any one of the preceding claims wherein the ester of 4-(2,3-epoxypropoxy)-phenylacetic acid is reacted with isopropylamine under conditions where the S configuration is substantially maintained to form the corresponding ester of 4-(2-hydroxy-3-isopropylaminopropoxy)-phenylacetic acid.

**6.** A process according to claim 5 wherein the ester is a methyl ester.

**7.** A process according to claim 5 wherein the ester of 4-(2-hydroxy-3-isopropylaminopropoxy)-phenylacetic acid is reacted with ammonia under conditions where the S configuration is substantially maintained to form 4-(2-hydroxy-3-isopropylaminopropoxy)phenylacetamide (atenolol).

8. A process according to claim 7 wherein the ester is a methyl ester.

9. A process according to claim 7 or 8 wherein the atenolol is reacted with a suitable acid to form a pharmaceutically acceptable salt.

**Revendications**

1. Procédé d'époxydation stéréosélective, qui comprend l'exposition d'un ester de l'acide 4-allyloxyphénylacétique à l'action d'une souche de Pseudomonas oleovorans ayant l'aptitude à l'époxydation stéréosélective de manière à produire l'ester correspondant de l'acide 4-(2,3-époxypropoxy)phénylacétique qui a en proportion prépondérante la configuration S.

2. Procédé suivant la revendication 1, dans lequel l'ester de l'acide époxypropoxyphénylacétique a pour plus de 80% en poids la configuration S.

3. Procédé suivant la revendication 2, dans lequel l'ester de l'acide époxypropoxyphénylacétique a pour plus de 90% en poids de la configuration S.

4. Procédé suivant la revendication 1, dans lequel le micro-organisme est Pseudomonas oleovorans - (ATCC 29347) ou un mutant ou variant de celui-ci.

5. Procédé suivant l'une quelconque des revendications précédentes, dans lequel l'ester de l'acide 4-(2,3-époxypropoxy)phénylacétique est mis à réagir avec l'isopropylamine dans des conditions où la configuration S est sensiblement conservée pour former l'ester correspondant de l'acide 4-(2-hydroxy-3-isopropylaminopropoxy)phénylacétique.

6. Procédé suivant la revendication 5, dans lequel l'ester est l'ester méthylique.

7. Procédé suivant la revendication 5, dans lequel l'ester de l'acide 4-(2-hydroxy-3-isopropylaminopropoxy)phénylacétique est mis à réagir avec l'ammoniac dans des conditions où la configuration S est sensiblement conservée pour former le 4-(2-hydroxy-3-isopropylaminopropoxy)phénylacétamide (aténolol).

8. Procédé suivant la revendication 7, dans lequel l'ester est l'ester méthylique.

9. Procédé suivant la revendication 7 ou 8, dans lequel l'aténolol est mis à réagir avec un acide approprié pour former un sel pharmaceutiquement acceptable.

**Patentansprüche**

1. Stereoselektives Epoxidationsverfahren, dadurch gekennzeichnet, dass man einen Ester der 4-Allyloxyphenylessigsäure der Einwirkung eines Pseudomonas oleovorans-Stammes aussetzt, der die Fähigkeit zur stereoselektiven Epoxidation hat, so dass der entsprechende Ester der 4-(2,3-Epoxypropoxy)-phenylessigsäure erzeugt wird, der überwiegend in der S-Konfiguration vorliegt.

2. Verfahren nach Anspruch 1, dadurch gekennzeichnet, dass mehr als 80 Gew.-% des Epoxypropoxyphenylessigsäureesters die S-Konfiguration aufweist.

3. Verfahren nach Anspruch 2, dadurch gekennzeichnet, dass mehr als 90 Gew.-% des Epoxypropoxyphenylessigsäureesters die S-Konfiguration aufweist.

4. Verfahren nach Anspruch 1, dadurch gekennzeichnet, dass der Mikroorganismus Pseudomonas oleovorans (ATCC 29347) oder eine Mutante oder Variante davon ist.

5. Verfahren nach einem der voranstehenden Ansprüche, dadurch gekennzeichnet, dass man den Ester der 4-(2,3-Epoxypropoxy)-phenylessigsäure unter solchen Bedingungen mit Isopropylamin umsetzt, dass die S-Konfiguration im wesentlichen beibehalten wird, um den entsprechenden Ester der 4-(2-Hydroxy-3-isopropylaminopropoxy)-phenylessigsäure zu bilden.

6. Verfahren nach Anspruch 5, dadurch gekennzeichnet, dass der Ester ein Methylester ist.

7. Verfahren nach Anspruch 5, dadurch gekennzeichnet, dass der Ester der 4-(2-Hydroxy-3-isopropylaminopropoxy)-phenylessigsäure unter solchen Bedingungen mit Ammoniak umgesetzt wird, dass die S-Konfiguration im wesentlichen beibehalten wird, um 4-(2-Hydroxy-3-isopropylaminopropoxy)-phenylacetamid (Atenolol) zu bilden.

8. Verfahren nach Anspruch 7, dadurch gekennzeichnet, dass der Ester ein Methylester ist.

9. Verfahren nach Anspruch 7 oder 8, dadurch gekennzeichnet, dass das Atenolol mit einer geeigneten Säure umgesetzt wird, um ein pharmazeutisch unbedenkliches Salz zu bilden.